# FASCICULE DE BREVET EUROPEEN

(11) **EP 1 474 100 B1**
(45) Date de publication et mention de la délivrance du brevet: **14.08.2013**
(21) Numéro de dépôt: 03718861.2
(22) Date de dépôt: 10.02.2003
(51) Int. Cl.: A61K 8/64, A61Q 19/08, A61Q 19/00, A61K 8/60

(54) **COMPOSITIONS COSMETIQUES OU DERMOPHARMACEUTIQUES POUR DIMINUER LES POCHES ET CERNES SOUS LES YEUX.**
KOSMETISCHE ODER DERMOPHARMAZEUTISCHE ZUSAMMENSETZUNGEN ZUR VERKLEINERUNG DER TRÄNENSÄCKE UND AUGENRINGE
COSMETIC OR DERMOPHARMACEUTICAL COMPOSITIONS WHICH ARE USED TO REDUCE BAGS AND CIRCLES UNDER THE EYES

(30) Priorité: 15.02.2002 FR 0201967
(43) Date de publication de la demande: 10.11.2004
(73) Titulaire: Sederma, 78612 Le Perray-en-Yvelines Cedex (FR)
(72) Inventeur: LINTNER, Karl, 78120 Rambouillet (FR)
(86) Numéro de dépôt international: PCT/FR2003/000441
(87) Numéro de publication internationale: WO 2003/068141

(56) Documents cités:
- WO-A-00/43417
- WO-A-01/64178
- DATABASE WPI Section Ch, Week 200064 Derwent Publications Ltd., London, GB; Class B05, AN 2000-658372 XP002216648 & JP 2000 219639 A (TOYO YAKUHIN KOGYO KK) , 8 août 2000 (2000-08-08)

## Description

La présente invention concerne de nouvelles compositions cosmétiques ou dermopharmaceutiques à usage topique pour prévenir les symptômes du relâchement cutané, plus particulièrement destinées au traitement des signes visibles du vieillissement et de la fatigue, notamment les « poches » et « cernes » sous les yeux, qui contiennent, dans un support cosmétiquement acceptable, une combinaison des trois composants suivants :
a) l'hespéridine ou les dérivés alkyles de l'hespéridine,
b) un dipeptide inhibiteur de l'enzyme A.C.E (angiotensine converting enzyme EC 3.4.15.1) choisi dans le groupe constitué de H-Val-Trp-OR₁, H-Val-Tyr-OR₁, H-His-Tyr-OR₁, H-Arg-Phe-OR₁, H-Tyr-Trp-OR₁, avec R₁ = H ou une chaîne alkyle de C₁ à C₂₄, préférentiellement soit C₁ à C₃, soit C₁₄ à C₁₈, ou R₁= NR₂R₃ avec R₂R₃ étant indépendamment l'un de l'autre = H ou une chaîne alkyle de 1 à 12 atomes de carbone, préférentiellement de 1 à 3 atomes de carbone, et
c) un oligopeptide R₂-Gly-Gln-Pro-Arg-OH avec R₂ =H ou une chaîne alcoyle de longueur carbonée entre C₂ et C₂₂.

Notre peau est la première image que chacun de nous offre au regard des autres. De tout temps, son aspect a été un sujet de préoccupation.

La connaissance actuelle de sa physiologie permet maintenant de proposer des solutions cosmétiques aux différents dysfonctionnements induits par les agressions extérieures ou par le vieillissement. Beaucoup de choses restent pourtant inconnues, mal comprises et mal maîtrisées.

Il en est ainsi, par exemple, du symptôme appelé communément « poches sous les yeux », « les yeux gonflés », (« puffy eyes « en Anglais). Cette condition de la peau sous les yeux, caractérisée par un certain gonflement, souvent des irritations, une peau flasque, des rougeurs, n'a pas une étiologie bien définie, bien décrite dans la littérature.

Si dans certains cas l'hypothyroîdisme est évoqué, dans d'autres on établit une connexion entre les phénomènes d'allergie qui conduirait à des rhinites, une rétention d'eau dans la partie du visage (nez, yeux, sinus et alentours). La fatigue, la fumée (chez les fumeurs) excessive et les phénomènes d'irritation cutanée associés contribuent également à l'apparition des « poches ». Un aperçu global de la littérature sur le sujet fait apparaître les connexions entre inflammation, oedème et rétention d'eau/balance sodique, drainage lymphatique perturbé et insuffisance veineuse couplée à une fragilité des capillaires. Biensûr, l'âge et le relâchement des tissus cutanés qui sont particulièrement minces au niveau du contour des yeux, jouent leur rôle aussi.

Bien que souvent négligée, la qualité de la circulation lymphatique est un des éléments majeurs qui permet à la peau de garder toutes ses qualités esthétiques et physiologiques (par exemple Lubach et al; (1996) Br; J. Dermatol. 135:733-737; Brand & Braathen (1996) Dermatology 196:283-288). Dans l'organisme, le système lymphatique joue de multiples rôles complexes qui couvrent des domaines comme la nutrition ou - l'immunologie.

En ce qui concerne cette demande de brevet, comme approche physiologique théorique, on peut considérer schématiquement que le système lymphatique est un système de drainage qui régule les différents liquides physiologiques en collectant les excès de liquide extracellulaire et son contenu pour en favoriser le retour dans la circulation sanguine (environ 3 litres par jour).

Il se compose de trois éléments distincts : les capillaires situés dans les espaces extracellulaires, les ganglions et les vaisseaux.

C'est au niveau des capillaires que se fait la collecte des liquides extracellulaires. La paroi de ces capillaires est faite de cellules endothéliales qui se recouvrent mutuellement. Sous la pression du liquide extracellulaire, ces cellules qui se chevauchent basculent légèrement vers l'intérieur, à la manière de portes tournantes qui ne fonctionneraient que dans un sens. Le liquide qui pénètre dans le capillaire ne peut en ressortir. Ensuite, une succession de valves tout au long du système lymphatique empêche le reflux de la lymphe vers les capillaires. La lymphe est un liquide clair, dont la composition est proche de celle du sang à l'exception des globules rouges, qui transporte des lymphocytes, des nutriments, des hormones et des métabolites. La lymphe n'est pas pompée comme le sang mais elle circule dans le système lymphatique grâce à la compression des vaisseaux lymphatiques par les muscles qui l'entourent. La régulation de la circulation lymphatique est sous la dépendance de nombreux neuromédiateurs par le biais de récepteurs spécifiques, dont la bradykinine, qui agissent soit sur la fréquence soit sur l'intensité des *contractions* qui font progresser la lymphe par *péristaltisme* dans le système lymphatique. Il est donc possible de corriger pharmacologiquement les dysfonctionnements (fréquence et/ou intensité) de ce péristaltisme lymphatique (par exemple Yokoyama et Benoit (1996) Am. J. Physiol.270:G752-756).

De nombreuses compositions cosmétiques destinées à améliorer l'aspect de la peau du visage ont été proposées à ce jour. Les produits hydratants, les crèmes anti-rides, les lotions calmantes aux effets anti-irritants en font partie. A notre connaissance, aucune n'a adressé le problème des poches sous les yeux » de façon systématique et concertée, agissant à plusieurs niveaux des causes et symptômes à la fois.

La présente invention vise à résoudre le problème esthétique posé par ces « poches » en s'attaquant à la fois aux problèmes de rétention d'eau, d'inflammation, de fragilité vasculaire et du relâchement des tissus cutanés par une combinaison synergique de trois catégories de substances actives, à savoir les hespéridines, les peptides inhibiteurs de l'enzyme convertissant l'angiotensine I en angiotensine II et les peptides immunomodulateurs, fragments d'immunoglobulines.

Ainsi, conformément à l'un des objets de la présente invention, il est maintenant proposé de nouvelles compositions cosmétiques et dermopharmaceutiques, en particulier de soins de la peau du visage, selon la revendication 1.

La présente invention a également pour objet l'utilisation de telles compositions comme, ou pour la fabrication de, compositions cosmétiques ou dermopharmaceutiques destinées aux soins du visage, en particulier le traitement des poches et cernes sous les yeux.

Un autre objet encore de la présente invention réside dans un procédé de traitement cosmétique de la peau du visage, en particulier des poches et cernes sous les yeux, qui consiste essentiellement à appliquer sur les zones concernées une quantité efficace d'une composition conforme à l'invention. D'autres caractéristiques, aspects et avantages de la présente invention apparaîtront à la lecture de la description détaillée qui va suivre.

### Description détaillée de l'invention :

L'hespéridine et ses dérivés, en particulier la hespéridine méthyle chalcone, sont des substances naturelles, ou modifiées synthétiquement à partir de la substance naturelle, et connues pour leur activité vitamine P. Le brevet FR 2183612 décrit leur utilisation pour le traitement de la fragilité capillaire. L'hespéridine méthyle chalcone est couramment employée dans des produits pharmaceutiques comme vasodilatateur et modificateur de la perméabilité capillaire.

Comme indiqué plus haut, le drainage lymphatique est sous la dépendance de certains neuromédiateurs, dont la bradykinine, un nonapetide circulant qui stimule l'évacuation lymphatique par l'activation des muscles entourant le réseau des vaisseaux. Le taux de bradykinine circulante est modulé par une enzyme, appelée « angiotensine converting enzyme » ou A.C.E. (EC 3.4.15.1) qui dégrade la bradykinine rapidement. Certains dipeptides, dont le dipeptide Val-Trp sont connus pour leur capacité d'inhiber cette enzyme de conversion (Dobbins D.E. et al (1990) Microcirc Endothelium Lymphatics 6:409-425). Ils sont donc capables d'améliorer le *drainage* lymphatique par l'augmentation de la circulation de la lymphe, via la régulation du taux de bradykinine circulant, influençant ainsi le péristaltisme lymphatique, dont l'effet aboutit à une peau *lavée de l'intérieur* des liquides et catabolites néfastes.

L'utilisation de ces dipeptides, en particulier de Val-Trp conduit à un autre effet bénéfique dans ce contexte. Nous avons dit que l'apparition des « poches » est aussi liée à des phénomènes de rétention d'eau, d'oedème local. Il est connu que le peptide angiotensine II (issu de la conversion de l'angiotensine I par l'enzyme A.C.E. citée) est un agent vasoconstricteur puissant, qui augmente en même temps la soif et le phénomène de rétention d'eau générale dans les vaisseaux. L'inhibition de l'A.C.E. par les dipeptides diminuera donc le taux circulant de l'angiotensine Il (seule forme active) en même temps qu'elle augmente le taux de bradykinine. Le peptide Val-Trp agit donc contre la rétention d'eau, l'imbalance sodique (également impliquée dans l'apparition des « poches ») et la formation locale des oedèmes associés.

Les inhibiteurs de l'enzyme de conversion de l'angiotensine connus ne peuvent pas tous être utilisés en cosmétique pour différentes raisons : tolérance ou toxicité, législation, compatibilité galénique, biodisponibilité. Il est donc évident que quelques modifications chimiques (hydrophile ou lyophilisation pour augmenter la solubilité ou la capacité de pénétration cutanée) peuvent être apportées aux molécules, pour autant qu'elles gardent leur activité initiale d'inhibition de l'enzyme de conversion.

Dans les cas particulier des dipeptides inhibiteurs de l'enzyme A.C.E. caractérisés par la structure H-X-Y-OH , avec X=Val, His, Trp, Arg ou Tyr, et Y=Tyr, Phe ou Trp, il est possible d'estérifier le groupe carboxyle avec un alcool, linéaire ou branché, hydroxylé ou non, conduisant aux composés H-X-Y-OR₁, soit par amidation avec une amine conduisant aux composés H-Val-Trp-ONR₂R₃, avec R₁=une chaîne alkyle de C₁ à C₂₄, préférentiellement soit C₁ à C₃, soit C₁₄ à C₁₈ et avec R₂R₃ étant indépendamment l'un de l'autre = H ou une chaîne alkyle de 1 à 12 atomes de carbone, préférentiellement de 1 à 3 atomes de carbone, sans que ces modifications changent en quoi que soit leur activité sur l'enzyme de conversion.

En troisième lieu, le peptide Gly-Gln-Pro-Arg et ses dérivés sont connus (WO0043417) pour leur activité anti-inflammatoire, en particulier quant à leur pouvoir d'inhiber la sécrétion de l'interleukine 6 (IL6) dans les cellules de la peau. Au cours des recherches de la présente invention, il a été découvert que ce peptide, en particulier sa forme dérivée Palmitoyl-Gly-Gln-Pro-Arg, possède des activités cosmétiques insoupçonnées, à savoir un effet raffermissant et restructurant de la peau du cou et du visage. Par son effet restructurant, il contribue également à une meilleure hydratation du tégument.

Ainsi l'objet de la présente demande est la découverte que l'utilisation cosmétique, à savoir par voie topique, et conjointe des trois composés d'activité complémentaire décrits, à savoir
a) l'hespéridine ou un dérivé alkyle de l'hespéridine
b) un dipeptide inhibiteur de l'enzyme A.C.E (angiotensine converting enzyme EC 3.4.15.1) choisi dans le groupe constitué de H-Val-Trp-OR₁, H-Val-Tyr-OR₁, H-His-Tyr-OR₁, H-Arg-Phe-OR₁ et H-Tyr-Trp-OR₁, avec R₁ = H ou une chaîne alkyle de C₁ à C₂₄, préférentiellement soit C₁ à C₃, soit C₁₄ à C₁₈, ou R₁= NR₂R₃ avec R₂R₃ étant indépendamment l'un de l'autre = H ou une chaîne alkyle de 1 à 12 atomes de carbone, préférentiellement de 1 à 3 atomes de carbone ; et
c) un oligopeptide R₂-Gly-Gln-Pro-Arg-OH avec R₂ =H ou une chaîne alcoyle de longueur carbonée entre C₂ et C₂₂.
permet de réduire de façon significative les symptômes appelés « poches et/ou cernes sous les yeux » . Il suffit pour cela d'incorporer les composés actifs à des concentrations suffisantes et efficaces dans des compositions cosmétique ou dermopharmaceutiques acceptables et d'en appliquer une quantité suffisante et efficace sur les parties du visage concernées, pendant une période allant de 2 semaines à 2 mois ou plus.

L'hespéridine ou ses dérivés peuvent être obtenus par extraction végétale à partir de nombreuses plantes *(citrus spp., ruscus spp.* et d'autres). Une forme particulièrement préférée pour la réalisation de l'invention est la hespéridine méthyle chalcone, également désigné par trimethylhespéridine chalcone. Elle est habituellement obtenue par alkylation de l'hespéridine naturelle en utilisant le dimethylsulfate en milieu alcalin (I. Sakieki, Nippon Kagaku Zasshi 79, 1958, 733), mais d'autres méthodes peuvent être employées par l'homme de l'art. Sont également inclus dans la présente demande d'autres dérivés alkyles de l'hespéridine naturelle ou synthétique.

L'hespéridine ou ses dérivés sont utilisés dans les composées cosmétiques conformes à l'invention à des concentrations variant entre 0.001% (p/p) et 50% (p/p), préférentiellement entre 0.01% (p/p) et 10% (p/p), plus particulièrement entre 0.05% (p/p) et 1% (p/p).

Les dipeptides inhibiteurs de l'enzyme A.C.E. H-X-Y-OH avec X=Val, His, Trp, Arg ou Tyr, et Y=Tyr, Phe ou Trp peuvent être obtenus soit par synthèse chimique classique (en phase hétérogène ou en phase homogène), soit par synthèse enzymatique (Kullman et al., J. Biol. Chem. 1980, 255, 8234) à partir des acides aminés constitutifs ou de leurs dérivés.

Pour améliorer la bio-disponibilité et le passage cutané des ces dipeptides on peut augmenter leur lipophilie soit par l'estérification du groupe carboxyle avec un alcool, linéaire ou branché, hydroxylée ou non, conduisant aux composés H-X-Y-OR₁, soit par amidation avec une amine conduisant aux composés H-X-Y-ONR₂R₃, avec R₁=une chaîne alkyle de C₁ à C₂₄, préférentiellement soit C₁ à C₃, soit C₁₄ à C₁₈ et avec R₂R₃ étant indépendamment l'un de l'autre = H ou une chaîne alkyle de 1 à 12 atomes de carbone, préférentiellement de 1 à 3 atomes de carbone.

Les dipeptides peuvent être obtenus également par fermentation d'une souche de bactéries, modifiées ou non par génie génétique, pour produire les séquences recherchées ou leurs différents fragments.

Enfin, les dipeptides peuvent être obtenus par extraction de protéines d'origine animale ou végétale, préférentiellement végétale (dont le blé et le riz où sa présence a été démontrée - Saito~1993), susceptibles de contenir ces séquences au sein de leur structure, suivie d'une hydrolyse contrôlée, enzymatique ou non, qui libère le fragment peptidique de séquence Val-Trp dans les plantes qui sont susceptibles de contenir ces séquences au sein de leur structure. L'hydrolysé ménagée permet de dégager ces fragments peptidiques.

Pour réaliser l'invention, il est possible, mais non nécessaire, d'extraire soit les protéines concernées d'abord et de les hydrolyser ensuite, soit d'effectuer l'hydrolyse d'abord sur un extrait brut et de purifier les fragments peptidiques ensuite. On peut également utiliser l'hydrolysat sans en extraire les fragments peptidiques en question, en s'assurant toutefois d'avoir arrêté la réaction enzymatique d'hydrolyse à temps et de doser la présence des peptides en question par des moyens analytiques appropriés (traçage par radioactivité, immunofluorescence ou immunoprécipitation avec des anticorps spécifiques, etc.).

D'autres procédés plus simples ou plus complexes, conduisant à des produits moins chers ou plus purs sont facilement envisageables par l'homme de l'art connaissant le métier d'extraction et de purification des protéines et peptides.

Les peptides inhibiteurs de l'enzyme A.C.E. ou leurs dérivés décrits ci-haut sont employés dans les compositions cosmétiques conformes à l'invention à des concentrations qui peuvent varier entre 0.0001% (p/p) et 1% (p/p), préférentiellement entre 0.001% (p/p) et 0.05% (p/p).

La troisième composante de l'invention est constituée par le peptide de séquence

R₂-Gly-Gln-Pro-Arg-OH

avec R₂ = H ou une chaîne alcoyle de longueur carbonée entre C₂ et C₂₂. Préférentiellement, R₂ est une chaîne alcoyle de longueur carbonée entre C₂ et C₂₂, en particulier la chaîne palmitoyle (C₁₆).

Ces peptides sont utilisés dans les compositions cosmétiques conformes à l'invention à des concentrations qui peuvent varier entre 0.0001 % (p/p) et 1% (p/p), préférentiellement entre 0.001% (p/p) et 0.05% (p/p).

Selon un mode de réalisation particulier de l'invention, les compositions cosmétiques contiennent l'hespéridine méthyle chalcone à des concentrations entre 0.05 et 1.0% (p/p), le peptide synthétique H-Val-Trp-OR₁ avec R₁ = H ou CH₃ ou -NH₂ à des concentrations variant entre 0.001 et 0.01% (p/p) et le peptide R₂-Gly-Gln-Pro-Arg-OH avec R₂= H ou alcoyle de longueur carbonée entre C₂ et C₁₈.

Les compositions sont par exemple des lotions, des laits ou des crèmes émollientes, des laits ou des crèmes pour les soins de la peau ou des cheveux, des crèmes, des lotions ou des laits démaquillants, des bases de fond de teint, des lotions, des laits ou des crèmes anti-solaires, des lotions, des laits ou des crèmes de bronzage artificiel, des crèmes ou des mousses de rasage, des lotions après-rasage, des shampoings, des rouges à lèvres, des mascaras ou des vernis à ongles.

Ces compositions peuvent également se présenter sous la forme de bâtons pour les lèvres destinés soit à les colorer, soit à éviter les gerçures, ou de produits de maquillage pour les yeux ou des fards et fonds de teint pour le visage.

Lorsque les compositions selon l'invention se présentent sous la forme d'émulsions de type eau-dans-l'huile ou huile-dans-l'eau, la phase grasse est essentiellement constituée d'un mélange de corps gras d'extraction ou de synthèse, avec au moins une huile, et éventuellement un autre corps gras. La phase grasse des émulsions peut constituer de 5 à 60% du poids total de l'émulsion.

La phase aqueuse desdites émulsions constitue de préférence de 30 à 85% du poids total de l'émulsion. La proportion de l'agent émulsionnant peut être comprise entre 1 et 20%, et de préférence entre 2 et 12% du poids total de l'émulsion. Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des lotions adoucissantes pour la peau ; les lotions huileuses peuvent en outre constituer des huiles moussantes contenant un tensioactif oléosoluble, des huiles pour le bain, etc.

Parmi les principaux adjuvants pouvant être présents dans les compositions selon l'invention, on peut citer les solvants organiques ou hydroglycoliques, y compris le MP-diol et les polyglycérines, les corps gras d'extraction ou de synthèse, les épaississants ioniques ou non ioniques, les adoucissants, opacifiants, stabilisants, émollients, les silicones, α- ou ß-hydroxyacides, les agents anti-mousse, les agents hydratants, les vitamines, parfums, conservateurs, séquestrants, colorants, les polymères gélifiants et viscosants, les tensioactifs et émulsifiants, les autres principes actifs hydro- ou liposolubles, les extraits végétaux, extraits tissulaires, extraits marins, filtres solaires, les antioxydants.

Les mono-ou poly-alcools plus particulièrement préférés sont choisis parmi l'éthanol, l'isopropanol, le propylène-glycol, le glycérol et le sorbitol.

A titre de corps gras, parmi les huiles minérales, on peut citer l'huile de vaseline ; parmi les huiles animales, les huiles de baleine, de requin, de phoque, de menhaden, de foie de flétan, de morue, de thon, de tortue, de pied de boeuf, de pied de cheval, de pied de mouton, de vison, de loutre, de marmotte, etc. ; parmi les huiles végétales, les huiles d'amande, de germes de blé, de jojoba, de sésame, de tournesol, de palme, de noix, de karité, de shoréa, de macadamia, de pépins de cassis et similaires.

Parmi les esters d'acides gras, on peut utiliser des esters d'acide en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acetylée, les huiles de silicone.

Parmi les cires, on peut citer la cire de Sipol, la cire de lanoline, la cire d'abeille, la cire de Candelila, la cire monocristalline, la cire de Carnauba, le spermaceti, le beurre de cacao, le beurre de karité, les cires de silicone, les huiles hydrogénées concrètes à 25°C, les sucroglycérides, les oléates, myristates, linoléates et stéarates de calcium, magnésium et aluminium. Parmi les alcools gras, on peut citer les alcools laurique, cétylique, myristique, stéarique, palmitique, oléique, et les alcools de GUERBET comme le 2-décyltétradécanol ou le 2-hexyldécanol. A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de glycérol, les alcools en C₁₂-C₁₈ comportant de 2 à 10 moles de glycérol. Il peut être aussi utile d'utiliser des épaississants tels que les dérivés de cellulose, les dérivés de d'acide polyacrylique, les gommes de guar ou de caroube ou la gomme de xanthane.

La composition selon l'invention peut également contenir des adjuvants habituellement utilisés en cosmétique ou en dermatologie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de lécithine dans l'eau en présence de tensioactif ou encore de dispersions aqueuses de sphérules lipidiques, constituées de couches moléculaires organisées enfermant une phase aqueuse encapsulée. On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétylsulfate de sodium, les acides gras sous forme de sels ou encore les lipides du type de ceux décrits dans les brevets français n° 2 315 991, 1 477 048 et 2 091 516 ou dans les demandes de brevet international WO 83/01 571 et WO 92/08685.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou non-ionique. A titre de groupements non-ioniques, on peut citer des groupements dérivés de polyéthylèneglycol. On peut aussi utiliser avantageusement comme lipides formant la phase lamellaire, des éthers de polyglycérol tel que ceux décrits dans les brevets français n°1 477 048 , 2 091 516, 2 465 780 et 2 482 128.

A titre de groupement ionique, on peut avantageusement utiliser un groupement dérivé d'un composé amphotère, anionique ou cationique. D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tel que le phosphatidylglycérol et le phosphatidylinositol.

Les substances actives peuvent être des substances ayant un intérêt pharmaceutique, alimentaire ou ayant une activité cosmétique. Lorsqu'elles sont hydrosolubles, elles peuvent être dissoutes de façon homogène ou elles sont dans la phase aqueuse encapsulée à l'intérieur des vésicules. Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone et ses sels ; des agents de brunissage artificiel tels que la dihydroxyacétone, l'érythrulose, le glycéraldéhyde, les y-dialdéhydes tels que l'aldéhyde tartique, ces composés étant éventuellement associés à des colorants ; des filtres solaires hydrosolubles ; des antiperspirants, des déodorants, des astringents, des produits rafraîchissants, toniques, cicatrisants, kératolytiques, dépilatoires, des eaux parfumées ; des extraits de tissus végétaux, tels que les polysaccharides; des colorants hydrosolubles ; des agents antipelliculaires ; des agents antiséborrhéiques, des oxydants tels que des agents de décoloration comme l'eau oxygénée ; des réducteurs tels que l'acide thioglycolique et ses sels.

On peut citer également les vitamines, les hormones, les enzymes, telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles peuvent se trouver incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

Les trois composants a) à c) objets de la présente demande (hespéridines, peptides inhibiteurs de l'enzyme A.C.E. et H-(AA)n-Pro-Arg-OH) peuvent être utilisés dans les compositions cosmétiques conformes à l'invention soit en ajout individuel, soit en tant que pré-mélange dans un excipient convenable, et utilisés sous forme de solution, de dispersion, d'émulsion, de pâte ou de poudre. Ils peuvent individuellement ou ensemble être véhiculés par des vecteurs cosmétiques tels que les macro-, micro- ou nanocapsules, des liposomes ou des chylomicrons, les macro-, micro- ou nanoparticules ou microéponges. Ils peuvent être également adsorbés sur des polymères organiques poudreux, les talcs, bentonites et autres supports minéraux.

Ils peuvent être utilisés dans une forme quelconque, ou sous une forme liée ou incorporée ou absorbée ou adsorbée à de macro-, micro- et nanopaficules, de macro-, micro- et nanocapsules, pour le traitement des textiles, fibres synthétiques ou naturelles, laines et tous matériaux susceptible d'être utilisé pour réaliser des vêtements et sous-vêtements de jour ou de nuit destinés à être en contact avec la peau tel que les collants, sous-vêtements, mouchoirs, lingettes, pour exercer leur effet cosmétique via ce contact textile/peau et permettre une délivrance topique continue.

A titre d'exemple illustrant l'invention, on cite quelques formules cosmétiques représentatives mais non limitatives de l'invention:

| **Exemple n° 1**: Gel | g/100g |
|---|---|
| Carbomer | 0,3 |
| Propylène glycol | 2,0 |
| Glycérine | 1,0 |
| Vaseline blanche | 1,5 |
| Cylomethicone | 6,0 |
| Crodacol C90 | 0,5 |
| Lubrajel^{R} MS | 10 |
| Triéthanolamine | 0,3 |
| H-Val-Trp-OH | 0,005 |
| Hespéridine Méthyle Chalcone | 0.250 |
| Palmitoyl-Gly-Gln-Pro-Arg-OH | 0.0015 |
| Eau, conservateurs, parfum | qsp 100 g |

Ce gel obtenu de façon extemporanée, peut être utilisé en application quotidienne sur la peau du visage, en particulier autour des yeux pour diminuer les infiltrations oedémateuses.

| **Exemple n°2: Crème** | g/100g |
|---|---|
| Volpo S2 | 2.4 |
| Volpo S20 | 2.6 |
| Prostéaryl 15 | 8.0 |
| Cire d'abeille | 0.5 |
| Stéaroxy dimethicone | 3.0 |
| Propylène glycol | 3.0 |
| Carbomer | 0.25 |
| Triéthanolamine | 0.25 |
| H-Val-Trp-OEt | 0,01 |
| Hespéridine | 0.050 |
| Ala-Gln-Pro-Arg-OH | 0.0005 |
| Eau, conservateurs, parfums | qsp 100 g |

Cette émulsion est utilisée pour hydrater, restructurer et calmer la peau du visage, en particulier sur les zones à peau fragile.

Les activités décrites au début de cette demande sont illustrées par les exemples suivants.

### Exemple n° 3: Activité du dipeptide Val-Trp pour inhiber l'A.C.E.

Le test est dérivé de la méthode Sigma Diagnostics N°305-UV.

L'enzyme de conversion (EC 3.4.15.1) décompose le tripeptide synthétique FAPGG en FAP + glycylglycine. La mesure de la variation de densité optique (Δ D.O.), à 340 nm est directement proportionnelle à la disparition du FAPGG , ce qui permet de quantifier l'activité de l'enzyme de conversion.

La recherche d'un effet inhibiteur d'une molécule se réalise alors selon le même principe , mais en présence de différentes concentrations des molécules à tester.

Le tableau suivant montre les résultats (moyenne, écart-type) obtenus après cinq essais différents:

| Peptide testé | Concentration | Δ D.O. | Ecart type | Inhibition |
|---|---|---|---|---|
| Aucun | - | 0,110 | 0.008 | |
| H-Val-Trp | 10 ppm | 0,080 | 0,010 | 27,3 % |
| N-Palmitoyl-Val-Trp | 10 ppm | 0,112 | 0,012 | 0% |
| H-Val-Trp-OMe | 10 ppm | 0,076 | 0,013 | 30.9 % |
| H-Val-Trp | 50 ppm | 0,018 | 0,008 | 83.4 % |
| N-Palmitoyl-Val-Trp | 50 ppm | 0,0107 | 0,009 | 2.7 % |
| H-Val-Trp-OMe | 50 ppm | 0,0106 | 0,010 | 90.2 % |

Ces résultats démontrent l'activité inhibitrice du peptide, la perte de cette activité quand on modifie sa structure chimique sur la partie N-terminale et son activité maintenue quand on l'estérifie.

### Exemple n° 4 : Activité anti-inflammatoire du peptide Palmitoyl-Gy-Gln-Pro-Arg-OH (Pal-GQPR) : Régulation du taux d'IL-6

Des fibroblastes et des kératinocytes humains normaux sont mis en culture dans un milieu classique, en l'absence ou en présence de différentes concentrations (10 à 45 ppm) du peptide Pal-GQPR. Après 24 heures, les cellules sont rincées dans le même milieu mais en l'absence de Pal-GQPR, puis, après avoir subi une irradiation de 35 mJ.cm²⁻¹, ces mêmes cellules sont remises en culture pour 48 heures, en présence ou en l'absence du Pal-GQPR, aux mêmes concentrations que celles de la période de culture initiale. Le dosage de l'IL-6 cellulaire est alors réalisé au moyen d'un kit Elisa standard.

Les deux tableaux suivants indiquent les résultats observés sur 5 expérimentations différentes réalisées avec le peptide mentionné en entête du tableau, les résultats (moyennes, σ) étant exprimés en pg.ml⁻¹ .20.000 cellules⁻¹.

**Table - 1**

| **N-Palmitoyl-Gly-Gln-Pro-Arg (ppm)** | | | | | | |
|---|---|---|---|---|---|---|
| | | **0** | **10** | **15** | **30** | **45** |
| **Fibroblastes** | ***Non irradiés*** | **23,1 *± 5,0*** | **22,2 *± 3,8*** | **18,4 *± 2,5*** | **180,0 *± 0,2*** | **17,0 *± 2,2*** |
| | ***Irradiés*** | **108,1 *± 25,0*** | **94,7 *± 11,2*** | **75,5 *± 3,2*** | **71,8 *± 10,0*** | **56,2 *± 8,0*** |
| **Kératinocytes** | ***Non irradiés*** | **0.071 *± 0,01*** | **0,055 *±0,01*** | **0,053 *± 0,01*** | **0,038 *± 0,01*** | **0,036 *± 0,01*** |
| | ***Irradiés*** | **0,752 *±0,01*** | **0,519 *± 0,15*** | **0,490 *±0,24*** | **0,376 *± 0,09*** | **0,325 *±0,05*** |

Dans les conditions « témoin» (absence de peptide au cours de l'expérimentation), les données de la littérature sont donc confirmées en ce qui concerne la surproduction d'IL-6 par des cellules exposées aux UV puisque l'on observe sur les fibroblastes et sur les kératinocytes, des augmentations du taux basal de respectivement 368 % (108,1 *vs* 23,1) et 959 % (0,752 *vs* 0,071).

A partir de ces données, il est évident que le peptide Pal-GQPR présente deux activités importantes, et ceci sur les deux types de cellules testées:
- *Diminution du taux basal d'IL-6:* Toutes les concentrations testées produisent une diminution du taux basal d'IL-6 avec, par exemple, une activité maximale pour la concentration maximale testée, égale à 26,4% sur les fibroblastes et 49,2 % sur les kératinocytes.
- *Diminution de la surproduction d'IL-6 lors d'une imadiation par les UV:* Toutes les concentrations testées produisent une diminution du taux observé dans les cellules non traitées, par exemple, avec une activité maximale pour la concentration maximale testée, égale à - 48,0 % sur les fibroblastes et - 56,8 % sur les kératinocytes.

Enfin, il est important de noter que les effets décrits ci-dessus sont parfaitement dépendants de la concentration de produit utilisé, ce qui renforce la notion de spécificité de l'action du peptide.

Parallèlement, pour s'assurer que la diminution des concentrations observées n'était pas due à une mortalité induite par le peptide lui-même, la viabilité des cellules a été vérifiée au moyen du test classique au MTT. Les résultats ont prouvé qu'à la concentration maximale testée (45 ppm), le peptide testé ne présentait aucune cytotoxicité sur le système étudié.

Le même type de résultats a été obtenu avec les autres séquences testées dans les mêmes conditions.

### Exemple n° 5 : Activité anti-poches

21 personnes (âge moyen 51 ans) participent à un test d'utilisation d'une crème contenant les trois composantes actives objets de la demande :
La formule suivante a été testée :

| Composant | % (p/p) |
|---|---|
| Eau | qsp 100 |
| Carbomer Ultrez 10 | 0.20 |
| Glycérine | 5.00 |
| Hydroxypropylcellulose | 0.20 |
| Pemulen TR2 (Goodrich) | 0.20 |
| Crodamol CAP (Croda) | 6.00 |
| Polysorbate 20 | 0.50 |
| Hespéridine méthyle chalcone | 0.15 |
| H-Val-Trp-OH | 0.003 |
| Palmitoyl-Gly-Gln-Pro-Arg-OH | 0.001 |
| Parfum, conservateurs | qsp |

Les « poches » sous les yeux sont évalués par auto-évaluation/questionnaire et par la technique de « projection de franges d'interférence » qui permet de mesurer les variations de la surface du visage avec une précision de 1/100 mm. Le produit est appliqué pendent 56 jours sur les zones concernées , 2 fois par jour. Les mesures sont faites à J0 et J56 . En résumé, il résulte de l'étude une diminution mesurable des « poches » qui peut atteindre 1 mm en profondeur. Par ailleurs, une majorité des panélistes confirme l'effet « anti-poches » dans le questionnaire d'auto évaluation.

## Revendications

1. Composition cosmétique ou dermopharmaceutique pour usage topique **caractérisée en ce qu'**elle contient, dans un support cosmétiquement acceptable, une combinaison des trois composants suivants :
a) l'hespéridine ou un dérivé alkyle de l'hespéridine ;
b) un dipeptide inhibiteur de l'enzyme A.C.E (angiotensine converting enzyme EC 3.4.15.1) choisi dans le groupe constitué de H-Val-Trp-OR₁, H-Val-Tyr-OR₁, H-His-Tyr-OR₁, H-Arg-Phe-OR₁ et H-Tyr-Trp-OR₁, avec R₁ = H ou une chaîne alkyle de C₁ à C₂₄, ou R₁=NR₂R₃ avec R₂R₃ étant indépendamment l'un de l'autre H ou une chaîne alkyle de 1 à 12 atomes de carbone ; et
c) un oligopeptide R₂-Gly-Gln-Pro-Arg-OH avec R₂ = H ou une chaîne alcoyle de longueur carbonée entre C₂ et C₂₂.

2. Composition selon la revendication 1, **caractérisée en ce que** R₁ est une chaîne alkyle de C₁ à C₃ ou due C₁₄ à C₁₈.

3. Composition selon la revendication 1 ou 2, **caractérisée en ce que** le dérivé alkyle de l'hespéridine est l'hespéridine méthyle chalcone.

4. Composition selon l'une des revendications 1 à 3, **caractérisée en ce que** le dipeptide est le H-Val-Trp-OH.

5. Composition selon l'une des revendications 1 à 4, **caractérisée en ce que** l'oligopeptide est le H-Gly-Gln-Pro-Arg-OH ou le Palmitoyl-Gly-Gln-Pro-Arg-OH.

6. Composition selon l'une des revendications 1 à 5, **caractérisée en ce que** la concentration en hespéridine ou en dérivé alkyle de l'hespéridine varie entre 0.01 % (p/p) et 10% (p/p).

7. Composition selon la revendication 6, **caractérisée en ce que** la concentration en hespéridine ou en dérivé alkyle de l'hespéridine varie entre 0.05% (p/p) et 1% (p/p).

8. Composition selon l'une des revendications 1 à 7, **caractérisée en ce que** la concentration en dipeptide inhibiteur de l'enzyme A.C.E. varie entre 0.0001 % (p/p) et 1% (p/p).

9. Composition selon la revendication 8, **caractérisée en ce que** la concentration en dipeptide varie entre 0.001% (p/p) et 0.05% (p/p).

10. Composition selon l'une des revendications 1 à 9, **caractérisée en ce que** la concentration en oligopeptide varie entre 0.0001% (p/p) et 1% (P/P).

11. Composition selon la revendication 10, **caractérisée en ce que** la concentration en oligopeptide varie entre 0.001% (p/p) et 0.05% (p/p).

12. Composition selon l'une des revendications 1 à 11, **caractérisée en ce qu'**elle contient outre les composants nommés a) à c) de la revendication 1 au moins un autre ingrédient habituellement utilisé en cosmétique dans un mélange cosmétiquement acceptable choisi parmi les catégories suivantes : solvants organiques ou hydroglycoliques, corps gras d'extraction ou de synthèse, épaississants ioniques ou non ioniques, adoucissants, opacifiants, stabilisants, émolliants, silicones, α-hydroxyacides, agents anti-mousse, agents hydratants, vitamines, parfums, conservateurs, séquestrants, colorants, polymères gélifiants et viscosants, tensioactifs et émulsifiants, autres principes actifs hydro- ou liposolubles, extraits végétaux, extraits tissulaires, extraits marins, filtres solaires, antioxydants.

13. Composition selon l'une des revendications 1 à 12 pour une utilisation comme produit.ou ingrédient cosmétique dans le traitement de la peau du visage.

14. Composition selon la revendication 13 pour une utilisation comme produit ou ingrédient cosmétique dans le traitement des « poches » sous les yeux.

15. Composition selon l'une des revendications précédentes 1 à 14 sous une forme liée ou incorporée ou absorbée ou adsorbée à de macro-, micro- et nanoparticules, de macro-, micro- et nanocapsules, dans les textiles, fibres synthétiques ou naturelles, laines et tout matériaux susceptibles d'être utilisé pour réaliser des vêtements et sous-vêtements de jour ou de nuit, directement au contact de la peau ou de des cheveux pour en permettre une délivrance topique continue.

## Patentansprüche

1. Kosmetische oder dermopharmazeutische Zusammensetzung für den topischen Einsatz, **dadurch gekennzeichnet, dass** sie in einem kosmetisch annehmbaren Träger eine Kombination aus den drei folgenden Komponenten enthält:
a) Hesperidin oder ein Alkylderivat des Hesperidins;
b) ein Dipeptid mit Inhibitor-Wirkung gegen das A.C.E. (angiotensine converting enzyme EC 3.4.15.1) aus der Gruppe bestehend aus H-Val-Trp-OR₁, H-Val-Tyr-OR₁, H-His-Tyr-OR₁, H-Arg-Phe-OR₁ und H-Tyr-Trp-OR₁, wobei R₁ = H oder einer C₁- bis C₂₄-Alkylkette, oder R₁ = NR₂R₃, wobei R₂R₃ unabhängig voneinander H oder eine Alkylkette von 1-12 Kohlenstoffatomen sind; und
c) ein Oligopeptid R₂-Gly-Gln-Pro-Arg-OH mit R₂ = H oder einer Alkylkette mit Kohlenstoffkettenlänge C₂ bis C₂₂.

2. Zusammensetzung nach Anspruch 1, **dadurch gekennzeichnet, dass** R₁ eine C₁- bis C₃- oder C₁₄- bis C₁₈-Alkylkette ist.

3. Zusammensetzung nach Anspruch 1 oder 2, **dadurch gekennzeichnet, dass** das Alkylderivat des Hesperidins das Hesperidinmethylchalcon ist.

4. Zusammensetzung nach einem der Ansprüche 1 bis 3, **dadurch gekennzeichnet, dass** das Dipeptid das H-Val-Trp-OH ist.

5. Zusammensetzung nach einem der Ansprüche 1 bis 4, **dadurch gekennzeichnet, dass** das Oligopeptid das H-Gly-Gln-Pro-Arg-OH oder das Palmitoyl-Gly-Gln-Pro-Arg-OH ist.

6. Zusammensetzung nach einem der Ansprüche 1 bis 5, **dadurch gekennzeichnet, dass** die Hesperidin- oder die Hesperidin-Alkylderivat-Konzentration zwischen 0,01 % (w/w) und 10 % (w/w) liegt.

7. Zusammensetzung nach Anspruch 6, **dadurch gekennzeichnet, dass** die Hesperidin- oder die Hesperidin-Alkylderivat-Konzentration zwischen 0,05 % (w/w) und 1 % (w/w) liegt.

8. Zusammensetzung nach einem der Ansprüche 1 bis 7, **dadurch gekennzeichnet, dass** die Konzentration von Dipeptid mit A.C.E.-Inhibitor-Wirkung zwischen 0,0001 % (w/w) und 1 % (w/w) liegt.

9. Zusammensetzung nach Anspruch 8, **dadurch gekennzeichnet, dass** die Dipeptid-Konzentration zwischen 0,001 % (w/w) und 0,05 % (w/w) liegt.

10. Zusammensetzung nach einem der Ansprüche 1 bis 9, **dadurch gekennzeichnet, dass** die Oligopeptid-Konzentration zwischen 0,0001 % (w/w) und 1 % (w/w) liegt.

11. Zusammensetzung nach Anspruch 10, **dadurch gekennzeichnet, dass** die Oligopeptid-Konzentration zwischen 0,001 % (w/w) und 0,05 % (w/w) liegt.

12. Zusammensetzung nach einem der Ansprüche 1 bis 11, **dadurch gekennzeichnet, dass** sie außer den unter a) bis c) in Anspruch 1 genannten Komponenten mindestens einen weiteren Inhaltsstoff umfasst, der in der Kosmetik in einer kosmetisch annehmbaren Mischung aus folgenden Kategorien üblicherweise verwendet wird: organische oder Hydroglykol-Lösungsmittel, extrahierte bzw. synthetische Fettstoffe, ionische bzw. nichtionische Verdickungsmittel, Weichmacher, Trübungsmittel, Stabilisatoren, Emollients, Silikone, Alpha-Hydroxy-Säuren, Entschäumer, Feuchtigkeitsmittel, Vitamine, Düfte, Konservierungsstoffe, Sequestriermittel, Farbstoff, gelierende und verdickende Polymere, Tenside und Emulsionsmittel, weitere wasser- bzw. fettlösliche Wirkstoffe, Pflanzenextrakte, Gewebeextrakte, Meeresextrakte, Lichtschutzfilter, Antioxidationsmittel.

13. Zusammensetzung nach einem der Ansprüche 1 bis 12, verwendet als kosmetisches Erzeugnis bzw. als kosmetischer Inhaltsstoff in der Gesichtshautpflege.

14. Zusammensetzung nach Anspruch 13, verwendet als kosmetisches Erzeugnis bzw. als kosmetischer Inhaltsstoff in der Behandlung von "Rändern" unter den Augen.

15. Zusammensetzung nach einem der vorhergehenden Ansprüchen 1 bis 14, gebunden oder beigemischt oder absorbiert oder adsorbiert mit bzw. in Makro-, Mikro- bzw. Nanopartikeln, Makro-, Mikro- bzw. Nanokapseln, in Textilien, synthetischen oder natürlichen Fasern, in Wollen und jeglichen Materialien, die zur Herstellung von Kleidungstücken bzw. Unterwäsche für den Tag oder die Nacht mit direktem Haut- oder. Haarkontakt verwendet werden können, um eine durchgängige, topische Abgabe davon zu ermöglichen.

## Claims

1. A topical cosmetic or dermopharmaceutical composition **characterized in that** it comprises, in a cosmetically acceptable base, a combination of the three following constituents:
a) hesperidin or an alkyl derivative of hesperidin;
b) a dipeptide that inhibits the A.C.E. enzyme (angiotensin converting enzyme EC 3.4.15.1) chosen from the group consisting of H-Val-Trp-OR₁, H-Val-Tyr-OR₁, H-His-Tyr-OR₁, H-Arg-Phe-OR₁ and H-Tyr-Trp-OR₁, with R₁ = H or a C₁ to C₂₄ alkyl chain, or R₁ = NR₂R₃ with R₂R₃ being independently from each other H or an alkyl chain of 1 to 12 carbon atoms; and
c) an R₂-Gly-Gln-Pro-Arg-OH oligopeptide with R₂ = H or an alkoyl chain between 2 and 22 carbons in length.

2. Composition according to claim 1, **characterized in that** R₁ is a C₁ to C₃ or C₁₄ to C₁₈ alkyl chain.

3. Composition according to claim 1 or 2, **characterized in that** the alkyl derivative of hesperidin is hesperidin methyl chalcone.

4. Composition according to anyone of claims 1 to 3, **characterized in that** the dipeptide is H-Val-Trp-OH.

5. Composition according to anyone of claims 1 to 4, **characterized in that** the oligopeptide is H-Gly-Gln-Pro-Arg-OH or Palmitoyl-Gly-Gln-Pro-Arg-OH.

6. Composition according to anyone of claims 1 to 5, **characterized in that** the concentration of hesperidin or alkyl derivative of hesperidin ranges between 0.01 % (w/w) and 10% (w/w).

7. Composition according to claim 6, **characterized in that** the concentration of hesperidin or alkyl derivative of hesperidin ranges between 0.05% (w/w) and 1 % (w/w).

8. Composition according to anyone of claims 1 to 7, **characterized in that** the concentration of the dipeptide inhibitor of A.C.E. enzyme ranges between 0.0001 % (w/w) and 1 % (w/w).

9. Composition according to claim 8, **characterized in that** the dipeptide concentration ranges between 0.001 % (w/w) and 0.05% (w/w).

10. Composition according to anyone of claims 1 to 9, **characterized in that** the concentration of oligopeptide ranges between 0.0001 % (w/w) and 1% (w/w).

11. Composition according to claim 10, **characterized in that** the concentration of oligopeptide ranges between 0.001 % (w/w) and 0.05% (w/w).

12. Composition according to anyone of claims 1 to 11, **characterized in that** in addition to the constituents named a) to c) of claim 1, the composition comprises at least one other constituent usually utilized in cosmetic in a cosmetically acceptable blend, selected from the group consisting of organic or aqueous-glycolic solvents, fatty substances obtained by extraction or synthesis, ionic or non-ionic thickeners, softeners, opacifiers, stabilizers, emollients, silicones, a-hydroxy acids, antifoaming agents, moisturizing agents, vitamins, perfumes, preservatives, sequestrating agents, colouring agents, gel-forming and viscosity-increasing polymers, surfactants and emulsifiers, other water- or fat-soluble active principles, plant extracts, tissue extracts, marine extracts, sun filters, or antioxidants.

13. Composition according to anyone of claims 1 to 12 for use as a cosmetic product or ingredient in the treatment of facial skin.

14. Composition according to claim 13 for use as a cosmetic product or ingredient in the treatment of under eye "bags".

15. Composition according to anyone of the preceding claims 1 to 14, in a form that is bound, incorporated, absorbed or adsorbed on macro-, micro- or nanoparticles, or macro-, micro- or nanocapsules, in textiles, natural or synthetic fibers, wools, or all materials capable of being used for clothing, or day or night underwear clothing, through direct contact with skin or hairs for a continuous topical delivery.
